(19) Europäisches Patentamt
European Patent Office
Office européen des brevets

(11) Numéro de publication: **0 593 917 A1**

# (12) DEMANDE DE BREVET EUROPEEN

(21) Numéro de dépôt: **93115155.9**

(22) Date de dépôt: **21.09.93**

(51) Int. Cl.⁵: **C07C 67/333**, C07C 69/75, C07C 327/14, C07C 61/22, A61K 7/46

(30) Priorité: **13.10.92 CH 3194/92**

(43) Date de publication de la demande: **27.04.94 Bulletin 94/17**

(84) Etats contractants désignés: **CH DE FR GB LI NL**

(71) Demandeur: **FIRMENICH SA**
**1, route des Jeunes**
**Case Postale 239**
**CH-1211 Genève 8(CH)**

(72) Inventeur: **Fehr, Charles, Dr.**
**6, chemin Ravoux**
**CH-1290 Versoix(CH)**
Inventeur: **Galindo, José**
**6, rue Carqueron**
**CH-1220 Les Avanchets(CH)**
Inventeur: **Stempf, Isabelle, Dr.**
**20, Domaine de l'Ile**
**F-67400 Illkirch-Graffenstaden(FR)**

(74) Mandataire: **Salvadori, Giuseppe, Dr. et al**
**c/o Firmenich S.A.**
**Case Postale 239**
**CH-1211 Genève 8 (CH)**

## (54) Procédé pour la préparation d'esters et thioesters optiquement actifs.

(57) Le procédé consiste en le traitement d'un énolate de formule

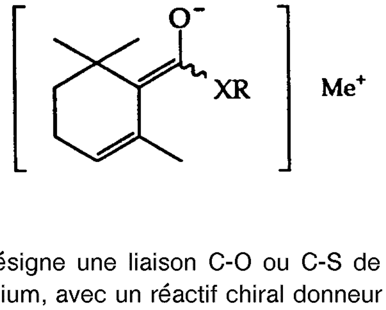

(II)

dans laquelle la ligne ondulée désigne une liaison C-O ou C-S de configuration cis ou trans, et Me définit un métal alcalin, de préférence le lithium, avec un réactif chiral donneur de protons.

Le procédé de l'invention permet l'obtention d'esters et thioesters optiquement actifs utiles pour la préparation de composés intéressant l'industrie des arômes et de la parfumerie ainsi que l'industrie pharmaceutique.

L'invention a trait également à l'utilisation de certains esters de l'acide cyclogéranique optiquement actifs à titre d'ingrédients parfumants.

EP 0 593 917 A1

La présente invention a trait au domaine de la synthèse organique. En particulier, elle concerne un procédé de préparation d'esters et thioesters optiquement actifs de structure nouvelle. Il s'agit de dérivés de l'acide cyclogéranique, ou cyclothiogéranique de formule

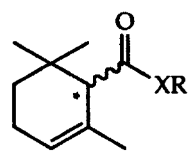

(I)

dans laquelle le symbole X sert à désigner un atome d'oxygène ou de soufre, R représente un reste aryle et l'astérisque définit un centre de chiralité de configuration (R) ou (S).

De tels esters se sont révélés être des auxiliaires précieux pour la synthèse de nombreux dérivés possédant une isomérie optique définie et dont les caractères organoleptiques en font des ingrédients parfumants ou aromatisants de choix. Il est apparu en effet que nombreux sont les composés qui, selon leur isomérie optique, présentent une différence de comportement marquée quant à leurs propriétés odorantes ou aromatisantes; certains composés pouvant être même inodores dans l'une des structures isomériques et odorants puissants dans l'autre.

Dans bien des cas, les méthodes de résolution des composés racémiques ne sont guère satisfaisantes, voire elles ne peuvent s'appliquer aux différents cas à l'examen, et, quoiqu'il en soit, elles ne sont pas économiquement avantageuses, permettant tout au plus l'isolation de 50% de l'isomère actif présent dans le mélange racémique.

C'est la raison pour laquelle il devient indispensable de pouvoir disposer de voies de synthèse énantiosélectives permettant la préparation d'isomères optiques purs ou, tout au moins, ayant une haute teneur en l'énantiomère désiré.

La présente invention apporte une solution à ce problème. Le procédé qu'elle définit est basé sur l'observation fortuite que les énolates des esters racémiques de formule

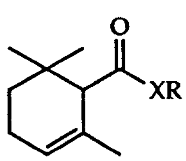

(±)-I

dans laquelle X et R ont le sens défini plus haut, pouvaient être soumis à une protonation énantiosélective aisée par traitement avec un réactif chiral donneur de protons, par exemple le (1R, 2S)-2-(N-méthyl-N-isopropylamino)-1-phénylpropan-1-ol ou son isomère (1S, 2R). On pouvait ainsi obtenir les esters ou thioesters (I) avec un haut degré d'énantiosélectivité. Or, ces esters ou thioesters se sont révélés être des matières premières chirales appropriées pour la préparation de divers composés optiquement actifs, utiles tout aussi bien pour l'industrie des arômes et de la parfumerie que pour l'industrie pharmaceutique. A titre d'exemple on peut citer l'α-damascone [voir demande de brevet européen N° 326'869], l'acide cyclogéranique, l'α-cyclocitral ou encore la forskoline.

Le procédé de l'invention est caractérisé en ce qu'on traite un énolate de formule

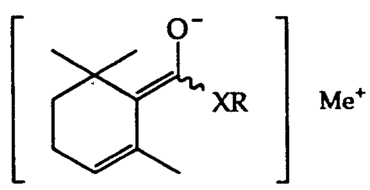

(II)

dans laquelle la ligne ondulée désigne une liaison C-O ou C-S de configuration cis ou trans, et Me définit un métal alcalin, de préférence le lithium, avec un réactif chiral donneur de protons constitué par une hydroxy-amine de formule

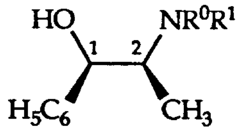

(1R,2S)

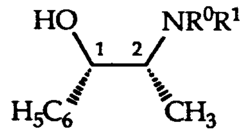

(1S,2R)

dans laquelle $R^0$ et $R^1$ pris séparemment définissent chacun un radical alkyle ou aralkyle, linéaire ou ramifié, ou l'un des deux représente un atome d'hydrogène et l'autre un radical alkyle tel que défini ci-dessus, ou pris conjointement ils représentent un radical cycloalkyle, et hydrolyse ensuite le mélange réactionnel pour fournir le (S)-, respectivement (R)-ester ou thioester désiré de formule

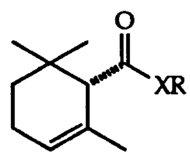

,

(S)-I

respectivement

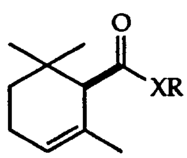

(R)-I

Comme indiqué plus haut, R sert à définir un radical aryle, substitué ou non. Il s'agit par exemple d'un phényle, substitué par exemple par un ou plusieurs restes alkyles comme le 2,6-diméthyl-phényle ou le p-chlorophényle, ou d'un naphtyle.

Les énolates de formule (II), utilisés comme produits de départ dans le procédé décrit plus haut, peuvent être obtenus aisément par traitement d'un ester de l'acide cyclogéranique ou cyclothiogéranique de formule

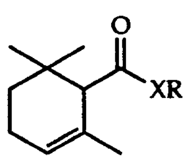

(±)-I

dans laquelle X et R ont le sens indiqué plus haut et R désigne de préférence le reste phényle, 2,6-diméthylphényle, p-chlorophényle ou naphtyle, au moyen d'une base forte tel un alkyl-lithium. A cet effet, le n-butyl-lithium est employé de préférence.

L'obtention de ces énolates peut se faire conformément à la méthode générale décrite dans la demande de brevet européen N° 326'869. Elle sera décrite dans le détail dans les exemples qui suivent.

Selon une variante, les thioénolates de départ peuvent être obtenus à partir d'un cétène de formule

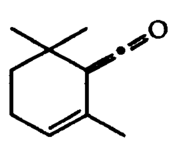

par traitement de ce dernier avec un sulphure lithié d'aryle en présence d'une source de protons chirale (R*OH). Une telle variante peut être illustrée par le schéma que voici :

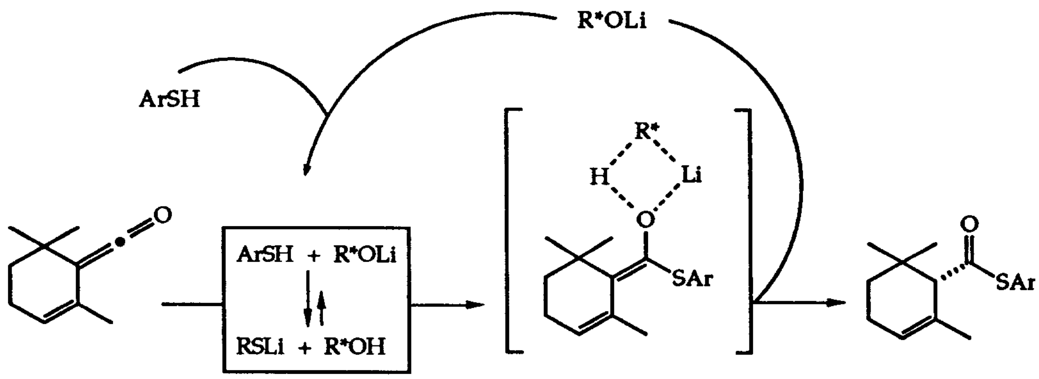


A titre de source de protons chiral on utilisera les mêmes dérivés de l'éphédrine que ceux mentionnés plus haut. La réaction procède par addition lente du sulphure d'aryle sur le cétène, formation du complexe constitué par l'énolate ainsi formé en combinaison avec le dérivé d'éphédrine choisi, suivie de la régénération de ce dernier avec formation graduelle du thioester désiré.

En tant que réactif chiral donneur de protons on peut employer la l-éphédrine, ou (1R, 2S)-2-(méthylamino)-1-phénylpropan-1-ol, le (1R, 2S)-2-(diméthylamino)-1-phénylpropan-1-ol, le (1R, 2S)-2-(isopropylamino)-1-phénylpropan-1-ol, le (1R, 2S)-2-(N-méthyl-N-isopropylamino)-1-phénylpropan-1-ol, ou leurs isomères (1S, 2R).

La première série de composés donnera accès aux thioesters ou esters (I) sous forme énantiomérique (S), tandis que l'utilisation d'un réactif chiral de configuration absolue (1S, 2R) permettra l'obtention de ces mêmes esters ou thioesters sous forme d'énantiomères (R), et ce avec un très haut niveau d'énantiosélectivité, proche en effet de 100%.

Ces composés peuvent être préparés à partir de l'éphédrine, produit de départ économique aisément disponible sur le marché.

C'est ainsi que le l-2-(N-méthyl-N-isopropylamino)-1-phénylpropan-1-ol peut être obtenu à partir de l-éphédrine par condensation au moyen d'acétone dans l'éthanol, suivie de réduction au moyen de $NaBH_4$, tandis que le 1-2-(isopropylamino)-1-phénylpropan-1-ol est obtenu par voie analogue à partir de l-noréphédrine suivant J. E. Saavedra [J. Org. Chem. 50, 2271 (1985)].

Les autres réactifs chiraux donneurs de protons à structure analogue peuvent être préparés par des méthodes similaires.

L'hydrolyse du mélange réactionnel obtenu peut s'effectuer au moyen d'une solution aqueuse acide, par exemple à l'aide d'un acide protique dilué, tel l'acide chlorhydrique ou sulfurique ou par traitement, soit avec une solution aqueuse de chlorure d'ammonium, soit avec un hydroxyde alcalin, de préférence à une température inférieure à la température ambiante.

Sous leur forme énantiomérique pure, les thioesters de l'invention représentent des entités chimiques nouvelles et à ce titre ils constituent également un des objets de l'invention.

Grâce au procédé de l'invention nous pouvons disposer ainsi de produits de départ dont le caractère chiral leur confère des propriétés utiles pour la préparation de composés finaux optiquement actifs.

A titre d'exemple nous indiquons ci-après le schéma de conversion du phényl-cyclothiogéraniate en l'acide cyclogéranique, α-damascone et α-cyclocitral, étant entendu que les procédés illustrés ici pour les dérivés (S) peuvent s'appliquer indifféremment à l'une ou l'autre des formes énantiomériques.

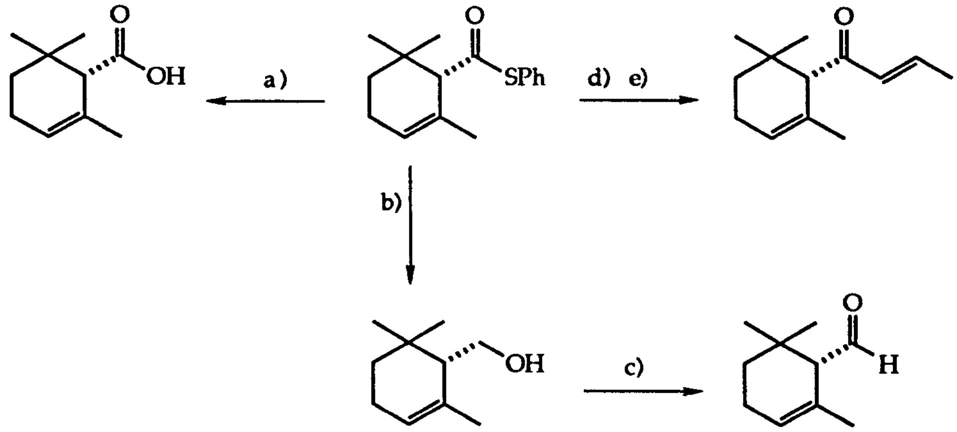


a) $H_2O_2$; LiOH, EtOH aq., 80°, 30min
b) $LiAlH_4$, $Et_2O$, 20°, 1h
c) $(COCl)_2$, $NEt_3$, DMSO, -70° → 20°, 2 h, puis $H_2O_2$ 70%, 20°, 30 min
d) chlorure d'allylmagnésium, LDA, THF, 35°, 1 h
e) TsOH (cat.), toluène, 20°, 15h.

Selon une variante du procédé de l'invention on utilisera une quantité de réactif chiral donneur de protons inférieure à la quantité stoechiométrique et la réaction de protonation énantiosélective pourra être complétée par l'adjonction d'un réactif achiral approprié donneur de protons.

La présente invention a également pour objet l'utilisation, à titre d'ingrédients parfumants, des esters de formule

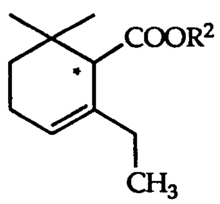

(III)

dans laquelle $R^2$ désigne un reste alkyle inférieur de $C_1$ à $C_3$ et l'astérisque désigne un centre de chiralité de configuration (R) ou (S), ainsi que de (+)-α-cyclogéraniate de méthyle.

Parmi les esters de formule (III) figurent également certains composés de structure nouvelle. Il s'agit tout particulièrement des énantiomères de formule

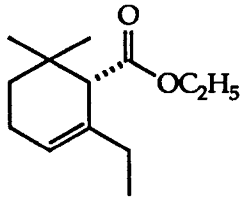

et

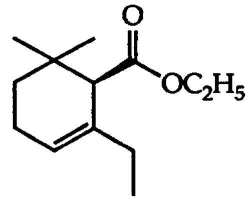

dont le composé racémique est décrit dans le brevet US N° 4,113,663.

Nous avons observé, tout comme dans le cas du composé de formule

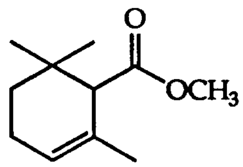 ,

qu'il existait une différence marquée, quant à leurs propriétés odorantes, entre le dérivé racémique et les énantiomères optiquement actifs correspondants et que de ce fait ces esters peuvent constituer des ingrédients parfumants de choix dont l'emploi se justifie de plein droit. Tout particulièrement nous avons pu établir que le (+)-α-cyclogéraniate de méthyle développe au mieux les caractères odorants plaisants propres à cet ester. Il possède en effet un caractère fleuri, damasconé avec un côté fruité-cassis qui peut se combiner harmonieusement avec bon nombre de constituants majeurs de la parfumerie, aussi bien de la parfumerie alcoolique que fonctionnelle.

De préférence il est adapté à la préparation de compositions parfumantes de type fruité, fleuri, rosé, boisé, épicé, chypre ou hespéridé par exemple. Le (+)-α-cyclogéraniate de méthyle est employé de préférence à raison de 0,1 à 5, voire 10% environ par rapport au poids de la composition dans laquelle il est incorporé. Des proportions supérieures à 10% peuvent être également considérées, en particulier lors de la préparation de bases ou coeurs pour parfums. Des proportions inférieures à 0,1% peuvent être également envisagées, notamment lors du parfumage de produits tels que savons, détergents ou produits cosmétiques par exemple.

Le (-)-α-cyclogéraniate de méthyle développe par contre un caractère métallique, camphré-menthé.

L'invention est illustrée de manière plus détaillée par les exemples suivants dans lesquels les températures sont indiquées en degrés centigrades et les abréviations ont le sens usuel dans l'art.

Exemple 1

(-)-(S)-2,6,6-triméthyl-cyclohex-2-ène-1-carbothioate de phényle

Une solution de 10 g (38,4 mmoles) de (±)-2,6,6-triméthyl-cyclohex-2-ène-1-carbothioate de phényle (= cyclothiogéraniate de phényle rac.) dans 250 ml de tétrahydrofuranne a été refroidie à -100/-110° (éther et azote liquide), puis traitée avec une solution de butyl-lithium 1,92 M dans l'hexane (30 ml; 57,7 mmoles) pendant 10 min à une température inférieure à -100°. Le mélange réactionnel a été ensuite maintenu sous agitation pendant 2 h à -105°/-100° et une solution de (-)-N-isopropyléphédrine [(1R, 2S)-2-(N-méthyl-N-isopropylamino)-1-phénylpropan-1-ol] (15,90 g; 76,8 mmoles) dans 20 ml de THF y a été ajoutée à -100° pendant 25 min. La température a été maintenue à -102°/-100° pendant 1 h puis augmentée à -10° (25 min) et le mélange a été ensuite versé dans une solution aqueuse à 5% de NaOH et extrait à l'éther.

La phase organique a été lavée successivement avec de l'eau, une solution aqueuse à 5% de HCl, puis encore avec de l'eau et une solution aqueuse de $NaHCO_3$, suivie d'une solution aqueuse de NaCl et séchée avec du $Na_2SO_4$. L'évaporation de l'excès de solvent et une distillation dans un four à boules (130°/6,6 Pa) ont permis l'obtention de 8,33 g (83%) de (-)-(S)-2,6,6-triméthyl-cyclohex-2-ène-1-carbothioate de phényle ayant une pureté de ≧ 97% et 99% ee; $[\alpha]^{20}_D$ = -421° ($CHCl_3$, c = 0,04).

Les phases aqueuses acides combinées ont été traitées avec une solution à 10% de $KOH/H_2O$, extraites à l'éther et distillées pour fournir 12,51 g (98%) de (-)-N-isopropyléphédrine.

Le carbothioate de phényle obtenu présentait les caractères analytiques suivants.

IR : 2970, 1705, 1480, 1440, 930 $cm^{-1}$

RMN($^1H$, 360MHz:) : 0,96(3H, s); 1,05(3H, s); 1,21(1H, m); 1,79(3H, large s); 1,91(1H, m); 1,98-2,18(2H, m); 2,86(1H, large s); 5,64(1H, large s); 7,38(5H, s) δppm.

SM : 151(13), 123(100), 109(12), 107(10), 91(9), 81(33).

Lorsqu'on remplace dans le procédé indiqué ci-dessus la (-)-N-isopropyléphédrine par son énantiomère (+)-N-isopropyléphédrine, on obtient le (+)-(R)-2,6,6-triméthyl-cyclohex-2-ène-1-carbothioate de phényle avec une pureté ≧ 95% et 99% ee.

$[\alpha]^{20}D$ = +406° ($CHCl_3$, c = 0,03).

Les thioesters ainsi obtenus ont été utilisés pour la préparation des composés suivants, selon le schéma réactionnel indiqué plus haut.

Acide (-)-(S)-2,6,6-triméthyl-cyclohex-2-énylcarboxylique :

$[\alpha]^{20}_D$ = -314° ($CHCl_3$, c = 0,07).

Acide (+)-(R)-2,6,6-triméthyl-cyclohex-2-énylcarboxylique :
$[\alpha]^{20}_D$ = +312° ($CHCl_3$, c = 0,05).
(-)-(S)-2,6,6-Triméthyl-cyclohex-2-ène-1-méthanol :
$[\alpha]^{20}_D$ = -133° ($CHCl_3$, c = 0,007).
(-)-(S)-α-Cyclocitral :
$[\alpha]^{20}_D$ = -743°.
(-)-(S)-α-Damascone :
$[\alpha]^{20}_D$ = -490°.

Exemple 2

(-)-(S)-2,6,6-Triméthyl-cyclohex-2-ène-1-carbothioate de phényle (conditions stoechiométriques)

Une solution de thiophénol (1,47 g; 13,33 mmoles) dans 10 ml de THF contenant un cristal de 1,10-phénanthroline a été traitée à environ 35-40° avec une solution 1,60 M de butyl-lithium (8,35 ml; 13,33 mmoles) jusqu'à ce que la solution devienne violette.
La solution a été décolorée par l'addition d'une goutte de (-)-isopropyléphédrine, diluée avec 40 ml de THF, refroidie à -55° et traitée ensuite avec 2 g (13,33 mmoles) de (±)-2,6,6-triméthyl-cyclohex-2-ényl-cétène. 2,76 g (13,33 mmoles) de (-)-isopropyléphédrine dans 5 ml de THF ont ensuite été ajoutés au mélange à -55° pendant 3 h au moyen d'une pompe à seringue et le tout a été versé dans une solution aqueuse à 5% de NaOH, puis extrait à l'éther.
La phase organique a été lavée avec de l'eau, une solution d'HCl à 5%, suivie d'une solution aqueuse saturée de $NaHCO_3$, une solution aqueuse saturée de NaCl et enfin séchée.
Par distillation dans un four à boules (130°/6,6 Pa) on a obtenu 2,90 g (rend. 84%) de (-)-(S)-2,6,6-triméthyl-cyclohex-2-ène-1-carbothioate de phényle ayant 95% ee.

Exemple 3

(-)-(S)-2,6,6-Triméthyl-cyclohex-2-ène-1-carbothioate de phényle (conditions catalytiques)

Une solution de 0,322 g (0,67 mmoles) de (-)-isopropyléphédrine, dans 5 ml de THF contenant un cristal de 1,10-phénanthroline a été traitée avec une solution de 1,60 M de butyl-lithium (0,42 ml; 0,67 mmoles) jusqu'à ce que la solution devienne violette brillante. Après addition de la première goutte de thiophénol (0,055 g; 0,05 ml) dans 0,2 ml de THF, la solution redevient incolore. Elle a été ensuite diluée avec 35 ml de THF, refroidie à -27° et traitée avec 2 g (13,33 mmoles) de 2,6,6-triméthyl-cyclohex-2-ényl-cétène. Au mélange réactionnel on a ensuite ajouté à -27°, à l'aide d'une pompe à seringue, en 3 h, une solution de thiophénol (1,35 g; 1,25 ml) dans 5 ml de THF. Une fois soumis aux traitements usuels, le mélange a fourni 2,88 g (rend. 86%) du thioester désiré; 89% ee.

Exemple 4

(-)-(S)-2,6,6-Triméthyl-cyclohex-2-ène-1-carbothioate de phényle

Une solution de (±)-2,6,6-triméthyl-cyclohex-2-ène-1-carbothioate de phényle (1,0 g ; 3,8 mmoles) dans 40 ml de THF a été refroidie à -100°/-110° et traitée avec une solution 1,54 M de butyl-lithium dans l'hexane (3,7 ml ; 5,7 mmoles). L'opération a lieu pendant 10 min à une température inférieure à -100°, puis le mélange a été maintenu sous agitation à -105°/-100° pendant 2 h. Une solution de (-)-N-isopropyléphédrine (0,398 g ; 1,9 mmoles) dans 3 ml de THF a été ajoutée au mélange (20 min) à -100° et à cette température on a additionné 0,777 g (58 mmoles) de phénylacétone dans 5 ml de THF (40 min). Après avoir été maintenue à environ -102°/-100° pendant 75 min, la température a été graduellement augmentée à -80° (40 min), puis, après réchauffement jusqu'à -10°, le mélange a été versé sous vigoureuse agitation dans une solution aqueuse à 5% de NaOH et extrait à l'éther. La phase organique a été lavée successivement avec de l'eau, une solution aqueuse à 5% d'HCl, encore de l'eau, une solution aqueuse saturée de $NaHCO_3$ et enfin une solution aqueuse de NaCl. Après séchage sur $Na_2SO_4$ et évaporation on a effectué une distillation dans un four à boules (T = 80-150°/4 x $10^2$ - 6,6 Pa) pour fournir 0,810 g (rend. 81%; pureté > 98%; ≧ 98% ee).

Exemple 5

(+)-(R)-α-Cyclogéraniate de méthyle

On a chargé sous azote un réacteur muni d'un agitateur par l'addition de 0,98 g (5,83 mmoles; ee ≧ 97%) d'acide (R)-α-cyclogéranique, 0,97 g (7 mmoles; 1,2 éq.) de $K_2CO_3$ anhydre, 0,44 ml (7 mmoles) d'iodure de méthyle et 25 ml d'acétone.

On a ensuite chauffé à reflux la suspension obtenue pendant 1,5 h et, après refroidissement à température ambiante, on l'a versée dans une solution aqueuse à 5% de NaOH. Le mélange de réaction a été extrait trois fois au pentane, trois fois à l'eau et enfin lavé avec une solution aqueuse saturée de NaCl.

Après séchage sur $Na_2SO_4$, suivi d'évaporation on a soumis le produit à distillation dans un four à boules [T = 100°/6,6 x $10^2$ Pa] pour fournir 0,92 g (rend. 87%) de (+)-(R)-α-cyclogéraniate de méthyle (ee ≧ 97%).

$[\alpha]^{20}_D$ = +308° ($CHCl_3$, c = 0,006).

Lorsque, dans le procédé ci-dessus, on remplace l'acide (R)-α-cyclogéranique par son isomère (S) correspondant on obtient le (-)-(S)-α-cyclogéraniate de méthyle (ee 99%).

$[\alpha]^{20}_D$ = -312° ($CHCl_3$, c = 0,006).

L'acide α-cyclogéranique, utilisé comme produit de départ, dans le procédé ci-dessus a été préparé ainsi.

Un mélange de 3,80 g (14,62 mmoles) de (S), ou respectivement (R)-2,6,6-triméthyl-cyclohex-2-ène-carbothioate de phényle, 30 ml d'éthanol, 1,84 g (43,85 mmoles) de LiOH dans 9 ml d'eau a été chauffé à reflux et 9,95 ml de $H_2O_2$ à 30% y ont été ajoutés dans un intervalle de 30 min. Le chauffage a été maintenu pendant 1 h, puis le mélange a été refroidi à 20°, acidifié avec de l'HCl aq. conc./glace et saturé avec du NaCl. On a ensuite extrait à l'éther et la phase organique a été lavée avec une solution de NaCl, 10% $NaHCO_3$ et à nouveau avec une solution aqueuse saturée de NaCl, puis séchée.

Par évaporation et distillation au four à boules (T = 140°/0,5 x $10^2$ Pa) on a obtenu les acides désirés.

(-)-(S) : $[\alpha]^{20}_D$ = -314° ($CHCl_3$, c = 0,03);

par cristallisation dans l'éther de pétrole on a obtenu un produit ayant $[\alpha]^{20}_D$ = -314°(EtOH, c = 0,07) ; 98% ee.

(+)-(R) : $[\alpha]^{20}_D$ = +312° ($CHCl_3$, c = 0,05) ; 98% ee.

Exemple 6

On a préparé une composition parfumante de base de type fleuri-herbacé en mélangeant les ingrédients suivants (parties en poids) :

| Ingrédients | Parties en poids |
|---|---|
| Bergamote synth. | 140 |
| Cedroxyde ® 1) | 40 |
| Citral | 60 |
| Citronellyle nitrile | 10 |
| Coumarine | 60 |
| Exaltex ® 2) | 50 |
| Essence de lavande | 60 |
| Lilial ® 3) | 180 |
| Lyral ® 4) | 60 |
| Mayol ® 5) | 110 |
| Essence de narcisse synth. | 20 |
| Essence de patchouli | 40 |
| Rose synth. | 100 |
| Vertofix ® 6) coeur | 40 |
| Total | 970 |

1) origine : Firmenich SA; 9,10-époxy-1,5,9-triméthyl-1,5-cyclododecadiène.
2) origine : Firmenich SA; cyclopentadécanolide.
3) origine : Givaudan-Roure; 2-méthyl-3-(4-ter-butyl-1-phényl)-propanal.
4) origine : IFF; 4-(4-hydroxy-4-méthylpentyl)-3-cyclohexène-1-carbaldéhyde.
5) origine : Firmenich SA; cis-7-p-menthanol.
6) origine : IFF; 1-(2,6,6,8-tétraméthyl[$5.3.1.0^{1,5}$]undéc-8-én-9-yle)-1-éthanone.

Lorsqu'on ajoute à 97 parties en poids de la composition de base ci-dessus, 3 parties en poids de (+)-α-cyclogéraniate de méthyle, le caractère naturel aromatique et fleuri de la composition s'en trouve renforcé. L'adjonction à la composition de base d'une même quantité de (-)-α-cyclogéraniate de méthyle produit par contre un effet négatif sur la note fleurie-fruitée qui disparait en faveur de l'apparition d'un caractère herbacé-métallique et camphré.

## Revendications

**1.** Procédé pour la préparation d'esters ou thioesters de formule

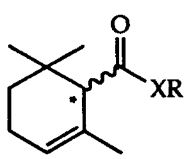

(I)

dans laquelle le symbole X sert à désigner un atome d'oxygène ou de soufre, R représente un reste aryle et l'astérisque définit un centre de chiralité de configuration (R) ou (S), caractérisé en ce qu'on traite un énolate de formule

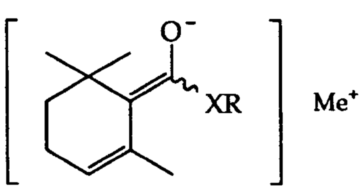

(II)

dans laquelle la ligne ondulée désigne une liaison C-O ou C-S de configuration cis ou trans, et Me définit un métal alcalin, de préférence le lithium, avec un réactif chiral donneur de protons constitué par une hydroxy-amine de formule

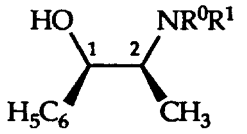
(1R,2S)

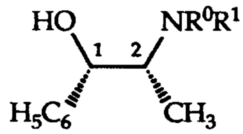
(1S,2R)

dans laquelle $R^0$ et $R^1$ pris séparemment définissent chacun un radical alkyle ou aralkyle, linéaire ou ramifié, ou l'un des deux représente un atome d'hydrogène et l'autre un radical alkyle tel que défini ci-dessus, ou pris conjointement ils représentent un radical cycloalkyle, et hydrolyse ensuite le mélange réactionnel pour fournir le (S)-, respectivement (R)-ester ou thioester désiré de formule

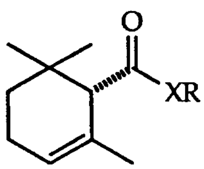
(S)-I

, respectivement

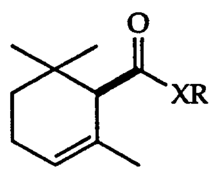
(R)-I

2. Procédé selon la revendication 1, caractérisé en ce qu'on utilise comme réactif chiral donneur de protons la (1R,2S)- ou (1S,2R)-2-(N-méthyl-N-isopropylamino)-1-phénylpropan-1-ol.

3. Procédé selon la revendication 1, caractérisé en ce qu'on utilise une quantité de réactif chiral donneur de protons inférieure à la quantité stoechiométrique et qu'on complète la réaction par l'addition d'un réactif achiral donneur de protons.

4. Procédé selon la revendication 1, caractérisé en ce que l'énolate de formule (I) est obtenu par traitement d'un ester ou thioester racémique de formule

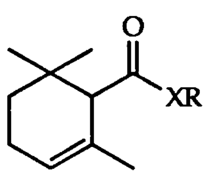
(±)-I

dans laquelle X et R ont le sens défini à la revendication 1, avec une base forte constituée par un alkyl-lithium.

5. Procédé selon la revendication 1, caractérisé en ce que l'énolate de formule (I) est obtenu à partir d'un cétène de formule

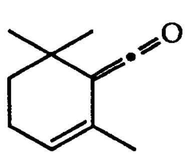

par traitement de ce dernier avec un sulphure lithié d'aryle en présence d'une source de protons chirale.

**6.** Thioesters de formule

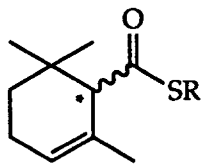

(Ib)

dans laquelle R représente un reste aryle et l'astérisque définit un centre de chiralité de configuration (R) ou (S).

**7.** Esters de formule

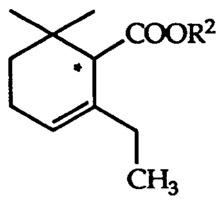

(III)

dans laquelle $R^2$ désigne un reste alkyle inférieur de $C_1$ à $C_3$ et l'astérisque désigne un centre de chiralité de configuration (R) ou (S).

**8.** Acides (-)-(S)-2,6,6-triméthyl-cyclohex-2-ène-carboxylique et (+)-(R)-2,6,6-triméthyl-cyclohex-2-ène-carboxylique obtenus par hydrolyse des thioesters selon la revendication 6.

**9.** Acides selon la revendication 8 obtenus par hydrolyse au moyen d'hydroperoxyde de lithium.

**10.** Utilisation d'esters selon la revendication 7 à titre d'ingrédients parfumants.

**11.** Utilisation de (+)-α-cyclogéraniate de méthyle à titre d'ingrédient parfumant.

Office européen des brevets

# RAPPORT DE RECHERCHE EUROPEENNE

Numero de la demande
EP 93 11 5155

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | JOURNAL OF ORGANIC CHEMISTRY. vol. 53, no. 8, 15 Avril 1988, EASTON US pages 1828 - 1830 CH.FEHR ET AL. 'REACTION OF ESTER ENOLATES WITH NUCLEOPHILES.STEREOCONTROLLED FORMATION OF KETONE AND ALDEHYDE ENOLATES' | 1 | C07C67/333<br>C07C69/75<br>C07C327/14<br>C07C61/22<br>A61K7/46 |
| X | HELVETICA CHIMICA ACTA. vol. 56, no. 7, 7 Novembre 1973, BASEL CH R.BUCHECKER AT AL. 'ABSOLUTE KONFIGURATION DER ENANTIOMEREN ALPHA-CYCLOGERANIUM-SAEUREN,ALPHA-GERANIALE,ALPHA-JONONE,GAMMA-JONONE ,ALPHA- UND EPSILON-CAROTINE'<br>* VERBINDUNGEN 1A UND 1B *<br>* page 2550 *<br>* page 2557, alinéa 3 * | 8 | |
| X | HELVETICA CHIMICA ACTA. vol. 52, no. 6, 22 Septembre 1969, BASEL CH pages 1729 - 1731 C.H.EUGSTER AT AL. 'BESTIMMUNG DER CHIRALITAET DER ENANTIOMEREN ALPHA-CYCLOGERANIUMSAEUREN,ALPHA-CYCLOGERANIALE,ALPHA-JONONE,GAMMA-JONONE,ALPHA-CAROTINE,EPSILON-CAROTINE UND VERWANDTE VERBINDUNGEN DURCH CHEMISCHE VERKNUEPFUNGSREAKTIONEN'<br>* FORMEL 1 *<br>* page 1730 * | 8 | **DOMAINES TECHNIQUES RECHERCHES (Int.Cl.5)**<br>C07C |
| A | EP-A-0 056 109 (FIRMENICH SA)<br>* revendications * | 1 | |

Le présent rapport a été établi pour toutes les revendications

| Lieu de la recherche | Date d'achèvement de la recherche | Examinateur |
|---|---|---|
| LA HAYE | 13 Janvier 1994 | KINZINGER, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)